# FASCICULE DE BREVET EUROPEEN

(11) **EP 0 581 619 B1**
(45) Date de publication et mention de la délivrance du brevet: **30.07.1997**
(21) Numéro de dépôt: 93400780.8
(22) Date de dépôt: 25.03.1993
(51) Int. Cl.: C10G 2/00, C07C 1/04, B01J 23/89

(54) **Procédé de conversion de gaz de synthèse en hydrocarbures avec un catalyseur à base de cobalt**
Verfahren zur Umwandlung von Synthesegas in Kohlenwasserstoffe auf Kobalt basierender Katalysator
Process for the conversion of synthesis gas into hydrocarbons with a cobalt based catalyst

(30) Priorité: 27.07.1992 FR 9209338
(43) Date de publication de la demande: 02.02.1994
(73) Titulaire: INSTITUT FRANCAIS DU PETROLE, 92502 Rueil-Malmaison (FR)
(72) Inventeur: Chaumette, Patrick, F-78380 Bougival (FR); Verdon, Catherine, F-92500 Rueil Malmaison (FR); Cruypelinck, Daniel, F-60440 Nanteuil le Haudoin (FR)

(56) Documents cités:
- EP-A- 0 142 887
- FR-A- 2 388 781

## Description

La présente invention est relative à un procédé de fabrication d'hydrocarbures à partir de mélanges CO-(CO₂)-H₂ c'est-à-dire de mélanges CO-H₂ comprenant éventuellement du CO₂ appelés gaz de synthèse. Elle concerne plus particulièrement l'utilisation d'un catalyseur permettant de réaliser la conversion du gaz de synthèse en un mélange d'hydrocarbures essentiellement C₅⁺ (c'est-à-dire possédant au moins 5 atomes de carbone par molécule) utilisables en tant que carburant ou combustible liquide.

Il est connu de l'homme du métier que le gaz de synthèse peut être converti en hydrocarbures en présence de catalyseurs contenant des métaux de transition.

Cette réaction, opérée à une température généralement comprise entre 150 et 350 °C et sous pression, est connue dans la littérature sous le nom de synthèse FISCHER-TROPSCH. Les catalyseurs habituellement utilisés pour la transformation de mélanges CO-(CO₂)-H₂ en hydrocarbures liquides ou gazeux comprennent généralement au moins un métal du groupe VIII tel que le fer, le ruthénium, le cobalt ou le nickel.

Les produits préparé par synthèse FISCHER-TROPSCH en présence de ces catalyseurs métalliques présentent une distribution très large en terme de poids moléculaire. Ainsi seulement une faible proportion des produits obtenus se situe dans la gamme des distillats moyens constitués par des fractions kérosène et gasoil, la (ou les) fraction(s) kérosène étant constituée(s) par un mélange d'hydrocarbures dont les points d'ébullition sont compris entre 140° et 300 °C, et la (ou les) fraction(s) gasoil étant constituée(s) par un mélange d'hydrocarbures dont les points d'ébullition sont compris entre 180° et 370 °C lors d'une distillation atmosphérique telle que réalisée par l'homme de l'art sur un brut pétrolier.

Des efforts importants ont été entrepris depuis 1973 afin d'améliorer le rendement en distillats moyens de procédés basés sur la conversion du gaz de synthèse. En particulier le cobalt, qui est connu en tant que constituant des catalyseurs Fischer-Tropsch depuis les premiers travaux de SABATIER et SENDERENS (J.Soc.Chem.lnd., 21, 504, 1902) et les brevets DE 29 787 (1913) et DE 295 202 (1914), a été à nouveau utilisé récemment en tant que constituant principal de formulations catalytiques améliorées.

Ces formulations permettent la synthèse d'hydrocarbures essentiellement paraffiniques et linéaires. Toutefois, une proportion importante de ces hydrocarbures est constituée de paraffines de hauts points d'ébullition, c'est-à-dire ayant des points d'ébullition situés au-delà du domaine des distillats moyens.

Il est alors avantageux de traiter ces hydrocarbures à haut point d'ébullition dans un procédé d'hydrocraquage habituellement utilisé dans le domaine du traitement des coupes lourdes issues d'un brut pétrolier, afin d'améliorer le rendement global en distillats moyens.

Parmi les formulations améliorées mentionnées précédemment, le brevet FR 2 388 781 revendique un procédé pour la préparation d'hydrocarbures utilisant un catalyseur contenant de 5 à 50 % en poids de un ou plusieurs métaux du groupe du fer parmi lesquels le cobalt et/ou de 0,1 à 5 % en poids de ruthénium et de 5 % à 50 % en poids de cuivre et/ou de zinc, ledit catalyseur étant préparé par imprégnation. Ledit procédé permet de préparer des hydrocarbures par réaction catalytique d'oxyde de carbone avec l'hydrogène, le rapport molaire H₂/CO étant inférieur à 1.

Le brevet FR 2 370 712 décrit l'utilisation dans un procédé de synthèse d'hydrocarbures d'un catalyseur comprenant 10 à 75 % poids d'un ou plusieurs métaux du groupe du fer ainsi que 1 à 50 % poids d'un ou plusieurs promoteurs parmi lesquels les métaux alcalins ou alcalino terreux, Ti, Zr, Al, Si, Cu, Ag, Ce, etc. Ce catalyseur est préparé par imprégnation d'un support poreux avec une ou plusieurs solutions aqueuses de sels des métaux du groupe du fer et des promoteurs.

Le brevet AU 46119/85 décrit un catalyseur actif en synthèse Fischer-Tropsch contenant (a) du cobalt ou un matériau à base de cobalt, (b) de la silice ou un précurseur de silice et (c) une base ou un matériau alcalin dans lequel le rapport a:b:c est compris dans le domaine 1:0,1-100:0,1-100.

Ledit catalyseur est préparé par synthèse hydrothermale sous pression et à une température comprise entre 50 °C et 500 °C.

La conversion du gaz de synthèse en carburants diesel est également décrite dans le brevet ZA 855 317. Ce brevet décrit des catalyseurs contenant essentiellement du cobalt et du ruthénium dispersés sur un support quelconque éventuellement additionné d'un promoteur choisi dans les groupes IIIB ou IVB, les oxydes d'actinides ou de lanthanides, ZrO₂ et TiO₂ étant préférés, ainsi que des catalyseurs contenant essentiellement du cobalt et un promoteur choisi dans le groupe constitué par Rh, Pt, Pd, Ir, Os, Ag, Au.

Ces formulations catalytiques sont préparées par imprégnation du support avec une solution organique contenant des sels solubles des différents éléments rentrant dans la composition de ces catalyseurs.

La demande de brevet français N° 91/07 634 décrit des catalyseurs contenant du cobalt, au moins un élément M additionnel choisi dans le groupe constitué par le molybdène et le tungstène et au moins un élément N choisi parmi différents éléments dont le ruthénium et le cuivre, ces éléments étant dispersés sur un support. Leur formulation se distingue de celle des catalyseurs selon l'invention décrits ci-après par la présence obligatoire de molybdène et/ou de tungstène qui conduit à la formation d'oléfines en plus grande proportion et/ou à des activités légèrement plus faibles.

Le brevet WO 85/04598 décrit un catalyseur de conversion du gaz de synthèse et le procédé de préparation dudit catalyseur comprenant une étape d'hydrolyse par exemple d'un composé du silicium ou de l'aluminium. Ce catalyseur contient au moins un métal choisi dans les groupes Vla et VIII de la classification périodique ; il ne contient pas d'élément du groupe Ib. Par ailleurs, les formulations préférés décrites dans ce brevet sont essentiellement à base de fer. L'hydrolyse des composés doit être menée pendant une durée suffisamment longue (1 à 12 heures) pour être pratiquement totale, une gélification rapide conduisant à un catalyseur dans lequel les divers éléments ne sont pas répartis de façon homogène. Le milieu d'hydrolyse et les espèces hydrolysées (ne contenant pas la matrice à base de silice et/ou alumine et les autres éléments) sont alors séparés par exemple par évaporation, puis le gel est traité thermiquement entre 100 °C et 600 °C.

Il a maintenant été trouvé une composition catalytique, conduisant, après réduction sous hydrogène, à une activité plus élevée en conversion du gaz de synthèse (mélange CO-(CO₂)-H₂) en un mélange d'hydrocarbures essentiellement linéaires et saturés contenant au moins 80 % poids par rapport à l'ensemble des hydrocarbures formés d'une coupe comprenant les hydrocarbures C₅⁺ et moins de 10 % poids d'oléfines dans ladite coupe C₅⁺.

Le catalyseur selon l'invention contient du cobalt, du cuivre et du ruthénium, et éventuellement au moins un élément P additionnel choisi dans le groupe constitué par les éléments des groupes Ia et IIa de la classification périodique des élements tels que les éléments Li, Na, K, Rb, Cs, Be, Mg, Ca, Sr et Ba, de préférence choisi dans le groupe formé par les éléments Na, K, Rb, Mg, Ca et Sr, l'ensemble de ces éléments étant dispersés sur un support. Il est préparé au moins partiellement par une technique d'imprégnation ou par une technique de gélification, de préférence totalement par une technique de gélification.

Le support utilisé comprend de la silice et/ou de l'alumine et éventuellement au moins un composé d'au moins un élément Q choisi dans le groupe formé par les éléments suivants : Si, Al, Ti, Zr, Sn, Zn.

La teneur en cobalt du catalyseur après calcination (traitement thermique entre 100 et 600 °C), exprimée en poids d'élément par rapport au poids de catalyseur, est habituellement comprise entre 1 et 60 % poids et préférentiellement entre 15 et 40 % poids.

Le cuivre, le ruthénium et le (ou les) élément(s) P additionnel(s) sont incorporés dans des proportions telles que :
- la teneur en ruthénium, exprimée en poids de ruthénium présent dans le catalyseur par rapport au poids de cobalt présent dans le catalyseur, est comprise entre 0,1 et 20 %, de préférence entre 1 et 5 %.
- la teneur en cuivre, exprimée en poids de cuivre présent dans le catalyseur par rapport au poids de cobalt présent dans le catalyseur, est comprise entre 0,1 et 10 %, de préférence entre 0,5 et 8 % et de manière encore plus préférée entre 1 et 8 %.
- la teneur en élément(s) P, exprimée en poids d'élément(s) P présent dans le catalyseur par rapport au poids de cobalt présent dans le catalyseur, est comprise entre 0 et 9 %, de préférence entre 0 et 7 %. Ainsi, selon la présence éventuelle respectivement d'un ou plusieurs élément(s) additionnel(s) P, ladite teneur est respectivement la teneur de l'élément P ou la somme des teneurs des éléments P.

Le catalyseur selon l'invention est préparé de préférence par une technique de gélification permettant d'incorporer le cobalt, le ruthénium, le cuivre, éventuellement au moins un composé d'élément P et éventuellement au moins un composé d'élément Q dans un alcogel formé à partir d'un composé hydrolysable du silicium et/ou de l'aluminium et éventuellement d'au moins un composé hydrolysable d'élément Q.

Cette réaction d'hydrolyse est menée rapidement, la durée de gélification étant inférieure ou égale à 30 mn et de préférence inférieure ou égale à 20 mn.

Les composés du cobalt, du ruthénium, du cuivre et éventuellement d'élément(s) P et/ou Q qu'il est possible d'utiliser dans cette technique de préparation sont par exemple choisis dans le groupe constitué par les halogénures, les nitrates, les acétates, les oxalates, les sulfates, les complexes formés avec l'acide oxalique et les oxalates, les complexes formés avec l'acide citrique et les citrates, les complexes formés avec l'acide tartrique et les tartrates, les complexes formés avec les acétyl acétonates, et tout autre dérivé inorganique ou organométallique contenant ces éléments.

Les composés hydrolysables du silicium et/ou de l'aluminium et éventuellement du silicium, de l'aluminium, du titane, du zirconium, de l'étain et du zinc utilisables dans cette technique de préparation sont par exemple choisis dans le groupe constitué par les alcoxydes, les carboxylates, et tout complexe organométallique soluble en solvant organique et hydrolysable.

Une technique de préparation préférée consiste à mélanger une solution A contenant un composé hydrolysable du silicium et/ou de l'aluminium et éventuellement au moins un composé hydrolysable d'un élément Q, de préférence un ou des alcoxydes, dissous dans un solvant organique, de préférence un alcool, et une solution aqueuse B contenant un composé de cobalt, un composé du cuivre, un composé du ruthénium et éventuellement un composé d'au moins un des éléments P et/ou Q, et contenant éventuellement un acide minéral qui accélère la gélification tel que par exemple l'acide nitrique, l'acide chlohydrique, l'acide sulfurique, l'acide phosphorique ou l'acide borique.

Le mélange des solutions A et B sous agitation à une température comprise entre 20 et 80 °C, conduit à l'obtention d'un alcogel massique qui est formé en moins de 30 mn, cet alcogel emprisonnant la quasi-totalité de la solution d'origine et donc la quasi-totalité des composés initialement dissous dans les solutions A et B.

L'alcogel ainsi obtenu est ensuite éventuellement séché à une température comprise entre 20 et 200 °C puis il est calciné, par exemple sous un courant d'air ou d'azote, jusqu'à une température comprise entre 100 °C et 600 °C.

Une autre méthode de préparation des catalyseurs selon l'invention consiste à préparer un hydrogel de silice et/ou d'alumine, contenant du cobalt, du cuivre, du ruthénium, et éventuellement au moins un élément P et/ou Q en titrant une solution aqueuse d'un ou plusieurs sels de silicium et/ou d'aluminium par une solution acide ou basique appropriée. Ainsi une solution aqueuse acide contenant par exemple un composé du silicium et/ou de l'aluminium choisi par exemple dans le groupe constitué par : l'acide silicique, le sulfate d'aluminium, le nitrate d'aluminium, le chlorure d'aluminium, peut être titrée par une solution basique contenant par exemple de l'hydroxyde de sodium, de l'hydroxyde d'ammonium, ou un aluminate et/ou silicate alcalin (voie 1).

Il est également possible de titrer une solution basique contenant, par exemple, un aluminate et/ou un silicate alcalin par une solution acide contenant par exemple de l'acide chlohydrique ou de l'acide nitrique (voie 2).

Les catalyseurs selon l'invention peuvent donc être préparés en dissolvant des composés du cobalt, du cuivre ou du ruthénium et éventuellement des éléments P et/ou Q dans l'une et/ou l'autre des solutions acides et/ou basiques et en réalisant l'une des titrations décrites ci-dessus (voie 1 ou 2) avec ces solutions acides et basiques. Cette titration sera préférentiellement effectuée en admettant simultanément les solutions acides et basiques dans un réacteur afin d'opérer la gélification à un pH constant compris entre 3,5 et 10, de préférence entre 5 et 10, la température de gélification étant comprise entre 20 et 90 °C et la durée de la gélification étant inférieure à 30 mn. Un mûrissement est ensuite éventuellement effectué en laissant évoluer le produit à une température comprise entre 20 et 90 °C en présence de la solution de gélification (eaux mères) ou d'une solution aqueuse contenant un sel basique, par exemple NaOH ou KOH, présentant un pH compris entre 10 et 12.

L'hydrogel est ensuite filtré, lavé par exemple avec de l'eau, éventuellement séché entre 20 et 200 °C, puis calciné, par exemple sous courant d'air ou d'azote, jusqu'à une température comprise entre 100 et 600 °C.

Une autre méthode de préparation préférée consiste à dissoudre les composés du cobalt, du cuivre, du ruthénium et éventuellement des éléments P et/ou Q dans de l'eau, puis à ajouter sous agitation une solution colloïdale de silice soit commerciale, soit préalablement préparée selon l'une des techniques de titration décrites auparavant et une solution d'acide, par exemple l'acide nitrique ou l'acide chlorhydrique pur ou dilué, en maintenant le pH du mélange entre 0,5 et 4, préférentiellement entre 1 et 3. Une fraction complémentaire de solution colloïdale de silice est ensuite ajoutée afin d'augmenter progressivement le pH de la solution jusqu'à une valeur comprise entre 3 et 8 unités pH, par exemple entre 4 et 8 unités pH, préférentiellement entre 4 et 7 unités pH, voire entre 5 et 7 unités pH. Après agitation pendant moins de 30 mn à une température comprise entre 5 et 90 °C, préfentiellement entre 20 et 60 °C, un gel de silice est formé qui contient les sels de cobalt, cuivre ruthénium et des éléments éventuels P et/ou Q. Ce gel est ensuite préférentiellement filtré, lavé par exemple avec de l'eau, éventuellement séché entre 20 et 200 °C, puis calciné, par exemple sous courant d'air ou d'azote, jusqu'à une température comprise entre 100 et 600 °C.

Il est également possible de préparer un alcogel ou un hydrogel contenant du silicium et/ou de l'aluminium et éventuellement les éléments P et/ou Q puis, avant traitement thermique, de remettre ce gel en suspension dans une solution aqueuse ou organique contenant du cobalt, du cuivre, du ruthénium et éventuellement les éléments P et/ou Q en excès par rapport aux quantités désirées dans le catalyseur final. Le pH de ladite solution est éventuellement ajusté au moyen d'un acide ou d'une base et est préférentiellement compris entre 4 et 8, la température étant comprise entre 25 et 100 °C. Le mélange gel + solution est agité pendant un temps suffisamment long, par exemple 30 mn à 5 heures, pour incorporer la quantité désirée de cobalt, cuivre, ruthénium et éventuellement des éléments P et/ou Q dans le gel. Le gel ainsi obtenu est éventuellement lavée, puis traité thermiquement entre 100 et 600 °C.

Le catalyseur peut éventuellement être mis en forme par tout procédé connu de l'homme de métier, par exemple par extrusion, coagulation en goutte, dragéification, pastillage ou spray-drying. Après cette étape de mise en forme, ledit catalyseur subit éventuellement une ultime activation thermique (séchage éventuel puis calcination) dans les conditions opératoires précitées.

Le catalyseur préparé selon les modes opératoires selon l'invention décrits précédemment est particulièrement bien adapté pour être utilisé dans les procédés de fabrication à partir d'un gaz de synthèse d'un mélange d'hydrocarbures essentiellement linéaires et saturés, contenant au moins 80 % en poids, par rapport à l'ensemble des hydrocarbures formés, d'une coupe comprenant des hydrocarbures C₅⁺ et moins de 10 % poids d'oléfines dans ladite coupe C₅⁺. La présente invention concerne donc également un procédé de synthèse d'hydrocarbures à partir de gaz de synthèse en présence d'un catalyseur préparé selon l'invention.

Les conditions de mise en oeuvre dudit catalyseur S pour la fabrication d'hydrocarbures sont habituellement les suivantes :

le catalyseur, chargé dans un réacteur, est tout d'abord préréduit par mise en contact avec un mélange de gaz inerte (azote par exemple) et d'au moins un composé réducteur (hydrogène et/ou monoxyde de carbone par exemple), le rapport molaire (composé réducteur) : (composé réducteur + gaz inerte) étant compris entre 0,001:1 et 1:1.

La préréduction est menée entre 150 °C et 600 °C, de préférence entre 200 °C et 500 °C, entre 0,1 MPa et 10 MPa et à une vitesse volumétrique horaire de 100 à 40 000 volumes de mélange par volume de catalyseur et par heure. Cette préréduction sera préférentiellement menée en phase liquide si, par la suite, la réaction de synthèse d'hydrocarbures se déroule en phase liquide. La phase liquide de la préréduction peut être constituée par au moins un hydrocarbure comprenant au moins 5 atomes de carbone par molécule.

Il peut également être avantageux de réoxyder le catalyseur sous un courant d'oxygène ou d'air dilué par de l'azote entre 30 °C et 650 °C, de préférence entre 50 °C et 450 °C à une pression comprise entre 0,1 MPa et 1 MPa à une vitesse volumétrique horaire de 100 à 40 000 volumes de catalyseur et par heure, puis de réaliser une nouvelle préréduction dans les conditions indiquées ci-avant.

La conversion du gaz de synthèse en hydrocarbures est ensuite opérée sous une pression totale habituellement comprise entre 0,1 MPa et 15 MPa, et de préférence entre 0,5 MPa et 10 MPa, la température étant généralement comprise 150 °C et 350 °C, et de préférence 170 °C et 300 °C.

La vitesse volumétrique horaire est habituellement comprise entre 100 et 10 000 volumes de gaz de synthèse par volume de catalyseur et par heure et de préférence entre 400 et 5 000 volumes de gaz de synthèse par volume de catalyseur et par heure, et le rapport H₂:CO dans le gaz de synthèse est habituellement compris entre 1:1 et 3:1, de préférence entre 1,2:1 et 2,5:1.

Le catalyseur peut être utilisé en poudre fine calibrée (10-700 µm habituellement) ou en particules de diamètre équivalent compris généralement entre 2 et 10 mm, en présence d'une phase gazeuse, ou d'une phase liquide (dans les conditions opératoires) et d'une phase gazeuse. La phase liquide peut être constituée par un ou plusieurs hydrocarbures ayant au moins 5, de préférence au moins 10, atomes de carbone par molécule.

Le catalyseur selon l'invention est particulièrement actif et stable dans la réaction de synthèse d'hydrocarbures à partir de gaz de synthèse ; il permet d'obtenir des hydrocarbures essentiellement paraffiniques, dont la fraction présentant les points d'ébulition les plus élevés peut être convertie avec un rendement élevé en distillats moyens (coupes gasoil et kérosène) par un procédé d'hydroconversion tel que l'hydrocraquage et/ou l'hydroisomérisation catalytique(s).

Les exemples suivants illustrent l'invention sans toutefois en limiter la portée.

### EXEMPLE 1 : Catalyseur A

Une solution 1 contenant 135 g d'orthosilicate d'éthyle (T.E.O.S.) dissous dans 50 ml d'éthanol et une solution 2 contenant 37 g de nitrate de cobalt hexahydraté, 0,31 g de trichlorure de ruthénium hexamine, 2,1 g de nitrate de cuivre trihydraté, 1,2 g de nitrate de potassium et 36,2 g d'acide nitrique concentré dissous dans 80 cm³ d'eau, sont mélangées sous forte agitation et à température ambiante.

L'hydrolyse du T.E.O.S. conduit au bout de 15 mn à la formation d'un gel massique contenant les sels de cobalt, de ruthénium, de cuivre et de potassium.

Le gel obtenu est séparé des eaux mères, séché à l'étuve entre 40 °C et 120 °C, puis calciné à 500 °C sous air - 50 g de catalyseur A sous forme oxyde et ayant la composition indiquée au tableau 1 sont alors obtenus.

### EXEMPLE 2 : Catalyseur B

A une solution contenant 40 g de nitrate de cobalt hexahydraté, 1 g de trichlorure de ruthénium hexamine et 0,30 g de nitrate de cuivre trihydraté dissous dans 50 ml d'eau sont ajoutés progressivement et simultanément 75 g de solution colloïdale de silice à 40 % poids de SiO₂ (LUDOX AS40) et 2 ml d'acide nitrique à 10 % de façon à maintenir le pH de la solution entre 1 et 2. La solution est maintenue sans agitation pendant 10 mn ; puis 25 g de LUDOX AS 40 sont ajoutés, le pH évoluant pour se stabiliser entre 5,5 et 6,5 unités pH. Après 12 mn, un gel de silice contenant les sels de cobalt, cuivre et ruthénium est formé.

Le gel obtenu est séparé des eaux mères par filtration, lavé à l'eau, séché à l'étuve entre 40 °C et 120 °C, puis calciné à 500 °C sous air. 40 g de catalyseur B sous forme oxyde et ayant la composition indiquée au tableau 1 sont alors obtenus.

### EXEMPLE 3 : Catalyseur C

La préparation du catalyseur C diffère de celle du catalyseur A en ce que l'on opère avec 62 g de nitrate de cobalt hexahydraté, 0,60 g de trichlorure de ruthénium dihydraté, 1,2 g de nitrate de cuivre dihydraté et 0,27 g de nitrate de strontium et sans ajouter d'acide nitrique à la solution 2, et en ce que la solution 1 ne contient que 112 g de T.E.O.S. dissous dans 40 ml d'éthanol.

Le catalyseur C sous forme oxyde ainsi obtenu à la composition indiquée au tableau 1.

### EXEMPLE 4 : Catalyseur D

La préparation du catalyseur D diffère de celle du catalyseur C en ce que la solution 1 contient 90 g de T.E.O.S. dissous dans 30 ml d'éthanol et la solution 2 contient 87 g de nitrate de cobalt hexahydraté, 1,1 g de trichlorure de ruthénium hexamine et 0,6 g de nitrate de cuivre trihydraté.

Le catalyseur D sous forme oxyde ainsi obtenu a la composition indiquée au tableau 1.

### EXEMPLE 5 : Catalyseur E

La préparation du catalyseur E diffère de celle du catalyseur C en ce que la solution 1 contient 100 g de T.E.O.S. et la solution 2 contient 20 g de nitrate d'aluminium à 9 molécules d'eau en lieu et place du nitrate de strontium.

Le catalyseur E sous forme oxyde ainsi obtenu à la composition indiquée au tableau 1.

### EXEMPLE 6 : Catalyseur F

La préparation du catalyseur F diffère de celle du catalyseur C en ce que la solution 1 contient 95 g de T.E.O.S. et la solution 2 contient 11,8 g de nitrate de zirconyle dihydraté en lieu et place du nitrate de strontium.

Le catalyseur F sous forme oxyde ainsi obtenu à la composition indiquée au tableau 1.

### EXEMPLE 7 : Catalyseur G

La préparation du catalyseur G diffère de celle du catalyseur B en ce que la solution contient 49 g de nitrate de cobalt hexahydraté, 0,5 g de trichlorure de ruthénium dihydraté, 0,95 g de nitrate de cuivre dihydraté et 54 g de solution aqueuse à 15 % poids de trichlorure de titane. D'autre part la quantité de solution colloïdale de silice ajoutée est de 90 g.

Le catalyseur G sous forme oxyde ainsi obtenu a la composition indiquée au tableau 1.

### EXEMPLE 8 (comparatif) : Catalyseur H

La préparation du catalyseur H diffère de celle du catalyseur D en ce que l'on introduit 87 g de nitrate de cobalt hexahydraté et 1,1 g de trichlorure de ruthénium hexaminé dans la solution 2.

Le catalyseur H sous forme oxyde ainsi obtenu à la composition indiquée au tableau 1.

### EXEMPLE 9 (comparatif) : Catalyseur I

La préparation du catalyseur I diffère de celle du catalyseur D en ce que l'on introduit 86,5 g de nitrate de cobalt hexahydraté et 0,60 g de nitrate de cuivre dihydraté dans la solution 2.

Le catalyseur I sous forme oxyde ainsi obtenu à la composition indiquée au tableau 1.

### EXEMPLE 10 (comparatif) : Catalyseur J

La préparation du catalyseur J diffère de celle du catalyseur D en ce que la solution 1 contient 85 g de T.E.O.S. et en ce que l'on introduit 86,5 g de nitrate de cobalt hexahydraté, 1,4 g d'heptamolybdate d'ammonium tétrahydraté, 1,1 g de trichlorure de ruthénium hexamine et 0,6 g de nitrate de cuivre trihydraté dans la solution 2.

Le catalyseur J sous forme oxyde ainsi obtenu à la composition indiquée au tableau 1.

### EXEMPLE 11 (comparatif) : Catalyseur K

Un support silice est imprégné (étape a) par une solution aqueuse de nitrate de cobalt hexahydraté d'un volume égal au volume poreux du support et contenant 20 % poids de cobalt par rapport au poids de silice, puis la solution est lentement évaporée à sec à 80 °C (étape b).

La silice imprégnée ainsi obtenue est ensuite séchée pendant 1 heure à 100 °C (étape c), et pendant 16 heures à 150 °C (étape d), puis calcinée pendant 3 heures à 500 °C (étape e).

On dépose ensuite 40 % poids de cobalt supplémentaire en 2 fois 20 % poids en répétant deux fois le protocole décrit dans les étapes a) à e) ; pour la deuxième imprégnation, le volume poreux considéré est celui du support imprégné par 20 % de cobalt. Enfin, on dépose 8% poids de cobalt, 2,1 % poids de ruthénium sous la forme de trichlorure de ruthénium dihydraté et 0,7 % poids de cuivre sous la forme de nitrate de cuivre dihydraté selon le protocole décrit dans les étapes a) à e), en considérant comme volume poreux celui du support imprégné par 40 % poids de cobalt.

Le catalyseur K sous forme oxyde ainsi obtenu a la composition indiquée au tableau 1.

### EXEMPLE 12 (Comparatif) : Catalyseur L

La préparation du catalyseur L diffère de celle du catalyseur D en ce que l'on ajoute en supplément 5,5 g de nitrate potassium dissous dans la solution 2. On obtient ainsi 3,9 % en poids de K par rapport au poids de catalyseur total, soit un rapport K:Co de 11.

Le catalyseur L sous forme oxyde ainsi obtenu a la composition indiquée au tableau 1.

### EXEMPLE 13 :

Les catalyseurs A à L, dont les préparations sont décrites dans les exemples 1 à 12, sont testés en phase gazeuse dans une unité pilote de type slurry fonctionnant en continu et opérant sur 100 cm³ de catalyseur.

Les catalyseurs A à L sont préalablement réduits in situ jusqu'à 240 °C par un mélange d'hydrogène et d'azote contenant 6 % d'hydrogène dans l'azote, puis par de l'hydrogène pur jusqu'à 350 °C, à la pression atmosphérique.

Les conditions de test des catalyseurs A à L sont les suivantes :
- température comprise entre 200 °C et 250 °C,
- pression 2MPa,
- vitesse volumique horaire (V.V.H.) 1000 h⁻¹,
- H₂:CO = 2:1,
- catalyseur en suspension dans un solvant paraffinique (coupe paraffinique C₁₅ - C₄₀) à la concentration de 10 % poids de catalyseur par rapport au poids de suspension.

La température de la suspension catalytique est réduite à 170 °C et le mélange hydrogène-azote est substitué par de l'azote pur.

La pression est alors portée à 2 MPa dans le réacteur et le gaz de synthèse (mélange hydrogène-monoxyde de carbone avec un rapport H₂:CO=2:1) est alors introduit progressivement de façon à obtenir la vitesse volumique horaire (V.V.H.) à 1000 h⁻¹.

Le débit d'azote est ensuite supprimé progressivement et la température ajustée à la valeur désirée (tableau 2, T=200 à 250 °C) avec une vitesse de montée en température de 6 °C/mn. Les performances catalytiques obtenues après stabilisation sous gaz de synthèse sont indiquées tableau 2.

Le tableau 2 montre que les catalyseurs selon l'invention permettent d'atteindre des conversions élevées du monoxyde de carbone (CO), indique également que plus de 80 % poids des hydrocarbures formés avec les catalyseurs selon l'invention, appartiennent à la coupe comprenant des hydrocarbures présentant au moins 5 atomes de carbone par molécule (hydrocarbures C₅⁺) et que ces catalyseurs conduisent à la formation de moins de 10 % poids d'oléfines dans ladite coupe C₅⁺. Une proportion importante des hydrocarbures formés se situe donc dans le domaine des distillats moyens (kérosène, gasoil) ou des paraffines solides à température ambiante (cires). La distribution des hydrocarbures obtenue est donc particulièrement bien adaptée à la préparation de distillats moyens, la fraction d'hydrocarbures présentant les points d'ébullition les plus élevés pouvant être convertie, avec un rendement élevé, en distillats moyens, par un procédé d'hydroconversion tel que l'hydrocraquage et/ou l'hydroisomérisation catalytiques.

Les exemples comparatifs du tableau 2 indiquent de plus que les catalyseurs H à K et plus particulièrement le catalyseur K, sont moins actifs, et que les catalyseurs H, I, J et L conduisent à la formation de quantités importantes d'oléfines dans la coupe C₅⁺. De plus, le catalyseur I conduit à la formation d'une quantité insuffisante d'hydrocarbures C₅⁺.

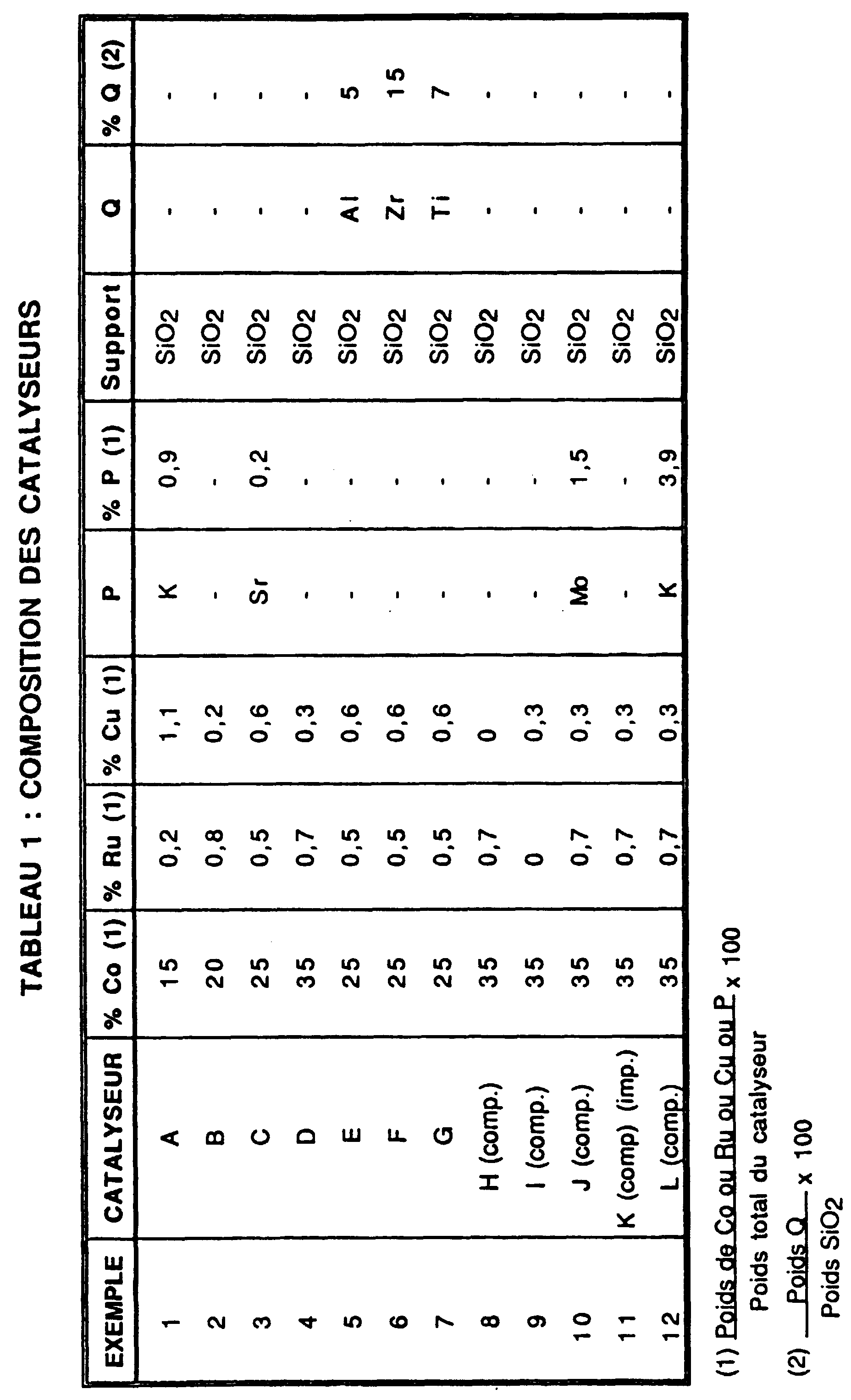

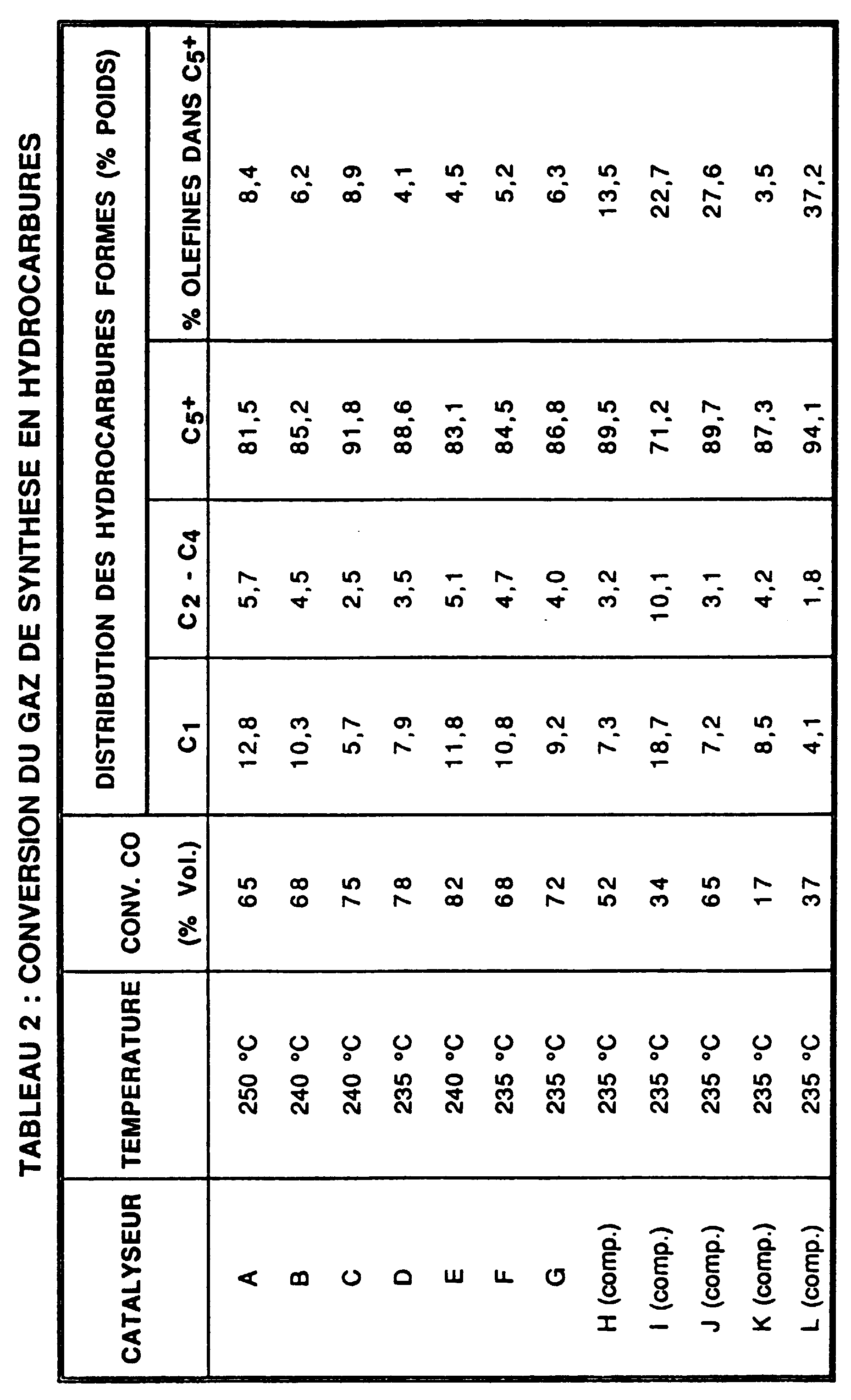

## Revendications

1. Procédé de conversion de gaz de synthèse en un mélange d'hydrocarbures essentiellement linéaires et saturés, caractérisé par l'emploi d'un catalyseur préparé par une technique de gélification et comprenant du cobalt, du cuivre et du ruthénium, le cobalt, le cuivre et le ruthénium étant dispersés sur un support comprenant au moins un oxyde d'un élément choisi dans le groupe formé par la silice et l'alumine, la teneur en cobalt, exprimée en poids de cobalt présent dans le catalyseur par rapport au poids de catalyseur, étant comprise entre 1 et 60 % poids, la teneur en ruthénium, exprimée en poids de ruthénium présent dans le catalyseur par rapport au poids de cobalt présent dans le catalyseur, étant comprise entre 0,1 et 20 %, et la teneur en cuivre, exprimée en poids de cuivre présent dans le catalyseur par rapport au poids de cobalt présent dans le catalyseur, étant comprise entre 0,1 et 10 %.

2. Procédé selon la revendication 1 dans lequel le catalyseur comprend en outre au moins un élément additionnel P, choisi dans le groupe formé par les éléments des groupes Ia et IIa de la classification périodique des éléments et dispersé sur le support, la teneur en élément(s) P, exprimée en poids d'élément(s) P présent(s) dans le catalyseur par rapport au poids de cobalt présent dans le catalyseur, étant comprise entre 0 et 9 %.

3. Procédé selon la revendication 2 dans lequel ladite teneur en élément(s) P est comprise entre 0 et 7 %.

4. Procédé selon l'une des revendications 1 à 3 dans lequel le support du catalyseur comprend en outre au moins un composé d'au moins un élément Q choisi dans le groupe formé par : Si, Al, Ti, Zr, Sn et Zn.

5. Procédé selon l'une des revendications 1 à 4 dans lequel ladite teneur en ruthénium est comprise entre 1 et 5 % et ladite teneur en cuivre est comprise entre 0,5 et 8 %.

6. Procédé selon l'une des revendications 1 à 5 dans lequel la durée de la préparation par gélification du catalyseur est inférieure ou égale à 30 mn.

7. Procédé selon l'une des revendications 1 à 6 tel que le mélange d'hydrocarbures essentiellement linéaires et saturés contient au moins 80 % poids, par rapport à l'ensemble des hydrocarbures formés, d'une coupe comprenant les hydrocarbures C₅⁺ et moins de 10 % en poids d'oléfines dans ladite coupe C₅⁺.

8. Procédé selon l'une des revendications 1 à 7 dans lequel le catalyseur est soumis à une préréduction avant utilisation, ladite préréduction du catalyseur étant effectuée par mise en contact avec un mélange de gaz inerte et d'au moins un composé réducteur en rapport molaire (composé réducteur) ; (composé réducteur + gaz inerte) compris entre 0,001 : 1 et 1 : 1, ledit composé réducteur étant choisi dans le groupe formé par l'hydrogène et le monoxyde de carbone, la préréduction étant menée entre 150 et 600 °C, entre 0,1 et 10 MPa et à une vitesse volumétrique horaire comprise entre 100 et 40 000 volumes de mélange par volume de catalyseur et par heure.

9. Procédé selon l'une des revendications 1 à 8 dans lequel on opère sous pression comprise entre 0,1 et 15 MPa, à une température comprise entre 150 et 350 °C, avec une vitesse volumétrique horaire comprise entre 100 et 10 000 volumes de gaz de synthèse par volume de catalyseur et par heure, et un rapport molaire H₂ : CO compris entre 1 : 1 et 3 : 1.

10. Procédé selon l'une des revendications 1 à 9 dans lequel ladite conversion est réalisée en présence d'une phase liquide comprenant au moins un hydrocarbure contenant au moins 5 atomes de carbone par molécule.

## Patentansprüche

1. Verfahren zur Umwandlung von Synthesegas in ein Gemisch aus im wesentlichen linearen und gesättigten Kohlenwasserstoffen, gekennzeichnet durch die Verwendung eines Katalysators, der durch eine Gelierungstechnik hergestellt wurde und Kobalt, Kupfer und Ruthenium umfaßt, wobei Kobalt, Kupfer und Ruthenium auf einem Träger dispergiert sind, der wenigstens ein Oxid eines Elementes umfaßt, das gewählt ist aus der Gruppe, die gebildet ist durch Siliciumoxid und Aluminiumoxid, wobei der Gehalt an Kobalt, ausgedrückt in Gewicht an im Katalysator enthaltenem Kobalt, bezogen auf das Gewicht des Katalysators, zwischen 1 und 60 Gew.-%, der Gehalt an Ruthenium, ausgedrückt in Gewicht an im Katalysator enthaltenem Ruthenium, bezogen auf das Gewicht von im Katalysator enthaltenem Kobalt, zwischen 0,1 und 20 % beträgt und der Gehalt an Kupfer, ausgedrückt in Gewicht an im Katalysator enthaltenem Kupfer, bezogen auf das Gewicht an im Katalysator vorhandenem Kobalt, zwischen 0,1 und 10 % liegt.

2. Verfahren nach Anspruch 1, bei dem der Katalysator im übrigen wenigstens ein Zusatzelement P umfaßt, das gewählt ist aus der Gruppe, die gebildet ist durch die Elemente der Gruppen Ia und IIa des Periodensystems der Elemente und auf dem Träger dispergiert ist, wobei der Gehalt an dem bzw. den Element(en) P, ausgedrückt in Gewicht an im Katalysator enthaltenen Elementen P, bezogen auf das Gewicht an im Katalysator vorhandenem Kobalt, zwischen 0 und 9 % liegt.

3. Verfahren nach Anspruch 2, bei dem dieser Gehalt an Element bzw. Elementen P zwischen 0 und 7 % liegt.

4. Verfahren nach einem der Ansprüche 1 bis 3, bei dem der Träger des Katalysators im übrigen wenigstens eine Verbindung wenigstens eines Elements Q umfaßt, das gewählt ist aus der Gruppe, die durch Si, Al, Ti, Zr, Sn und Zn gebildet ist.

5. Verfahren nach einem der Ansprüche 1 bis 4, bei dem dieser Gehalt an Ruthenium zwischen 1 und 5 % und dieser Gehalt an Kupfer zwischen 0,5 und 8 % liegt.

6. Verfahren nach einem der Ansprüche 1 bis 5, bei dem die Dauer der Herstellung durch Gelierung des Katalysators kleiner oder gleich 30 Minuten beträgt.

7. Verfahren nach einem der Ansprüche 1 bis 6, derart, daß das Gemisch an im wesentlichen linearen und gesättigten Kohlenwasserstoffen wenigstens 80 Gew.-% bezogen auf die Gesamtheit der Kohlenwasserstoffe beträgt, die aus einer Fraktion geformt sind, welche die C₅⁺ Kohlenwasserstoffe und wenigstens 10 Gew.-% Olefine in dieser C₅⁺ Fraktion umfaßt.

8. Verfahren nach einem der Ansprüche 1 bis 7, bei dem der Katalysator einer Vorreduktion vor der Verwendung ausgesetzt wird, wobei diese Vorreduktion des Katalysators durch Kontaktierung mit einem Gemisch eines inerten Gases und wenigstens einer reduzierenden Zusammensetzung im Molverhältnis wie folgt (reduzierende Zusammensetzung) vorgenommen wird:
(reduzierende Zusammensetzung + inertes Gas) zwischen 0,001:1 und 1:1, wobei die reduzierende Verbindung gewählt ist aus der Gruppe, welche geformt wird durch Wasserstoff und Kohlenmonoxid, die Vorreduktion zwischen 150 und 600°C zwischen 0,1 und 10 MPa und bei einer volumetrischen stündlichen Geschwindigkeit zwischen 100 und 40 000 Volumengemisch pro Volumenkatalysator und Stunde durchgeführt wird.

9. Verfahren nach einem der Ansprüche 1 bis 8, bei dem man unter Druck zwischen 0,1 und 15 MPa bei einer Temperatur zwischen 150 und 350°C bei einer stündlichen volumetrischen Geschwindigkeit zwischen 100 und 10 000 Volumensynthesegas pro Volumenkatalysator und Stunde und einem Molverhältnis H₂:CO zwischen 1:1 und 3:1 arbeitet.

10. Verfahren nach einem der Ansprüche 1 bis 9, bei dem diese Umwandlung in Anwesenheit einer flüssigen Phase realisiert wird, die wenigstens einen Kohlenwasserstoff umfaßt, der wenigstens 5 Atome Kohlenstoff pro Molekül enthält.

## Claims

1. Process for the conversion of synthesis gases into a mixture of essentially linear and saturated hydrocarbons, characterized by the use of a catalyst prepared by a gelling procedure and incorporating cobalt, copper and ruthenium, the cobalt, copper and ruthenium being dispersed on a support having at least one oxide of an element chosen from within the group formed by silica and alumina, the cobalt content expressed as cobalt weight present in the catalyst based on the catalyst weight, being between 1 and 60% by weight, the ruthenium content, expressed as ruthenium weight present in the catalyst based on the cobalt weight present in the catalyst, being between 0.1 and 20%, and the copper content, expressed as copper weight present in the catalyst based on the cobalt weight present in the catalyst, being between 0.1 and 10%.

2. Process according to claim 1, wherein the catalyst also comprises at least one additional element P, chosen from within the group formed by elements of groups Ia and IIa of the periodic classification of elements and dispersed on the support, the content of element or elements P, expressed by the weight of the element or elements P present in the catalyst, based on the cobalt weight present in the catalyst, being between 0 and 9%.

3. Process according to claim 2, wherein the said content of element or elements P is between 0 and 7%.

4. Process according to any one of the claims 1 to 3, wherein the catalyst support also comprises at least one compound of at least one element Q chosen from within the group formed by Si, Al, Ti, Zr, Sn and Zn.

5. Process according to any one of the claims 1 to 4, wherein the ruthenium content is between 1 and 5% and the copper content is between 0.5 and 8%.

6. Process according to any one of the claims 1 to 5, wherein the duration of the preparation by gelling of the catalyst is equal to or below 30 mn.

7. Process according to any one of the claims 1 to 6 such that the mixture of essentially linear and saturated hydrocarbons contains at least 80% by weight, based on all the hydrocarbons formed, of a fraction incorporating C₅⁺ hydrocarbons and less than 10% by weight olefins in said C₅⁺ fraction.

8. Process according to any one of the claims 1 to 7, wherein the catalyst undergoes a prereduction prior to use, said catalyst prereduction being carried out by contacting with a mixture of inert gases and at least one reducing compound in a molar ratio of [reducing compound: (reducing compound + inert gas)] between 0.001:1 and 1:1, said reducing compound being chosen from within the group formed by carbon monoxide and hydrogen, prereduction being performed at between 150 and 600°C, at between 0.1 and 10 MPa and a hourly volumetric rate between 100 and 40,000 volumes of mixture per volume of catalyst and per hour.

9. Process according to any one of the claims 1 to 8, wherein working takes place under a pressure between 0.1 and 15 MPa, at a temperature between 150 and 350°C, at a space velocity between 100 and 10,000 volumes of synthesis gas per volume of catalyst and per hour and with a H₂:CO molar ratio between 1:1 and 3:1.

10. Process according to any one of the claims 1 to 9, wherein the said conversion is performed in the presence of a liquid phase incorporating at least one hydrocarbon containing at least 5 carbon atoms per molecule.
